(19) Europäisches Patentamt
European Patent Office
Office européen des brevets

(11) **EP 3 814 307 B1**

(12) **FASCICULE DE BREVET EUROPEEN**

(45) Date de publication et mention de la délivrance du brevet:
**20.04.2022  Bulletin 2022/16**

(21) Numéro de dépôt: **19731299.4**

(22) Date de dépôt: **21.06.2019**

(51) Classification Internationale des Brevets (IPC):
**C07C 7/00** *(2006.01)*    **C07C 7/04** *(2006.01)*
**C07C 7/13** *(2006.01)*    **C07C 15/08** *(2006.01)*
**B01D 15/18** *(2006.01)*

(52) Classification Coopérative des Brevets (CPC):
(C-Sets disponibles)
**C07C 7/005; B01D 15/1828; C07C 7/04; C07C 7/13**
(Cont.)

(86) Numéro de dépôt international:
**PCT/EP2019/066445**

(87) Numéro de publication internationale:
**WO 2020/002142 (02.01.2020 Gazette 2020/01)**

(54) **PROCEDE DE PRODUCTION DE PARAXYLENE UTILISANT UNE ETAPE EN LIT MOBILE SIMULE, ET UNE ETAPE DE FRACTIONNEMENT DE DEUX FRACTIONS DANS UNE COLONNES DE 2 COUPES**

VERFAHREN ZUR HERSTELLUNG VON PARAXYLEN MIT EINEM SIMULIERTEN BEWEGTBETTSCHRITT UND VERFAHREN ZUR FRAKTIONIERUNG VON ZWEI FRAKTIONEN IN EINER ZWEIGETEILTEN KOLONNE

METHOD FOR PRODUCING PARAXYLENE USING A SIMULATED MOVING-BED STEP, AND A STEP OF FRACTIONATING TWO FRACTIONS IN A TWO-SECTION COLUMN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.06.2018  FR 1856049**

(43) Date de publication de la demande:
**05.05.2021  Bulletin 2021/18**

(73) Titulaire: **AXENS**
**92508 Rueil-Malmaison Cedex (FR)**

(72) Inventeurs:
• **PREVOST, Isabelle**
**92508 Rueil-Malmaison Cedex (FR)**
• **PIGOURIER, Jérôme**
**92508 Rueil-Malmaison Cedex (FR)**
• **HOTIER, Gérard**
**92508 Rueil-Malmaison Cedex (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**WO-A1-2013/089902    WO-A1-2016/133589**
**FR-A1- 2 844 790    FR-A1- 2 922 547**
**FR-B1- 2 844 790    US-B1- 6 407 303**

(52) Classification Coopérative des Brevets (CPC):
(Cont.)

C-Sets
**C07C 7/005;**
**C07C 7/04;**
**C07C 7/13**

**Description**

**DOMAINE TECHNIQUE**

Le paraxylène est principalement utilisé pour les productions d'acide téréphtalique et de résines polyéthylène téréphtalate, pour fournir des textiles synthétiques, des bouteilles, et plus généralement des matières plastiques.

La présente invention concerne un procédé d'obtention de paraxylène à haute pureté mettant en œuvre un enchainement spécifique d'étapes permettant de simplifier l'étape de fractionnement.

**ART ANTERIEUR**

La production de paraxylène haute pureté mettant en œuvre une étape de séparation par adsorption est bien connue de l'art antérieur. De manière industrielle, ladite étape est réalisée au sein d'un enchainement de procédés dit « boucle C8-aromatique » ou encore « boucle de xylène ». Cette « boucle C8-aromatique » inclut une étape d'élimination des composés lourds (c'est-à-dire contenant plus de 9 atomes de carbone, notés C9+) dans une colonne de distillation appelée « colonne des xylènes ».

Le flux de tête de cette colonne, qui contient les isomères en C8-aromatiques, est ensuite envoyé dans le procédé de séparation du paraxylène qui est généralement une étape de séparation par adsorption en lit mobile simulé.

L'extrait obtenu à l'issue de l'étape de séparation par adsorption en lit mobile simulé, qui contient le paraxylène est ensuite distillé au moyen d'une colonne d'extrait puis d'une colonne toluène, pour obtenir du paraxylène de haute pureté.

Le raffinat obtenu à l'issu de l'étape de séparation par adsorption en lit mobile simulé, riche en métaxylène, orthoxylène et éthylbenzène, après une étape d'élimination du désorbant par distillation, le mélange est mis en œuvre dans une étape d'isomérisation, permettant l'obtention d'un mélange dans lequel la proportion des xylènes (ortho-, méta-, para- xylènes) est pratiquement à l'équilibre thermodynamique, et la quantité d'éthylbenzène amoindrie. Ce mélange est à nouveau envoyé dans la « colonne des xylènes » avec la charge fraiche.

L'art antérieur propose de nombreuses variantes de ce schéma de base mettant en œuvre une ou plusieurs étape de séparation (par adsorption, cristallisation, distillation ou par membrane) et/ou une ou plusieurs étape d'isomérisation en phase gazeuse (convertissant l'éthylbenzène par isomérisation en xylènes ou par désalkylation en benzène), ou en phase liquide (ne convertissant pas l'éthylbenzène).

Le brevet FR2862638 décrit un procédé de production de paraxylène à partir d'une charge d'hydrocarbures, utilisant deux étapes de séparation en lit mobile simulé, et deux étapes d'isomérisation. L'inconvénient de ce procédé est de nécessiter deux étapes de séparation en lit mobile simulé qui entraine une augmentation importante du coût de production.

Les brevets FR 2844790 et FR2922547 décrivent un procédé d'obtention de paraxylène à partir d'une charge contenant des xylènes, de l'éthylbenzène et des hydrocarbures en C9+, ledit procédé comprenant- une unique étape A de séparation en lit mobile simulé de ladite charge, ladite étape étant mise en oeuvre avec une zéolithe comme adsorbant et un désorbant, permettant l'obtention d'au moins trois fractions, ■ une fraction Al comprenant un mélange de paraxylène et de désorbant, et ■ deux fractions A21, A22 comprenant de l'éthylbenzène (EB), de l'orthoxylène (OX) et du métaxylène (MX) et du désorbant. Ces deux documents décrivent des colonnes de distillation distinctes lors du traitement des raffinats A21 et A22.

L'inconvénient principal de ces variantes est de complexifier le procédé en mettant en œuvre une ou plusieurs étapes d'adsorption ou d'isomérisation. Cette complexité augment de façon conséquente les coûts d'investissements pour la mise en œuvre desdits procédée.

Dans le domaine de l'invention, l'homme du métier cherche constamment à limiter la consommation énergétique du complexe aromatique en conservant la quantité de paraxylène à haute pureté obtenu. En effet l'impact énergétique revêt une importance grandissante pour les opérateurs compte tenu des incitations croissantes à réduire l'empreinte carbone de leur unités.

De manière surprenante, la demanderesse a découvert que la combinaison dans un procédé d'obtention de paraxylène à haute pureté, d'une étape de séparation par adsorption en SMB produisant deux fractions contenant un mélange d'éthylbenzène (EB), métaxylène (MX),d'orthoxylène (OX), et de désorbant dans des proportions différentes, lesdites fractions étant engagées séparément dans une unique colonne de séparation par distillation permet avantageusement de faciliter l'étape de fractionnement du raffinat sans augmenter la complexité du procédé et sans modifier le rendement en Paraxylène.

Un autre avantage du procédé selon l'invention est de pouvoir être mise à profit dans des configurations de dégoulottage (« revamping ») d'une boucle xylènes. En effet, dans ce cas la même colonne de raffinat peut être réutiliser à condition de l'alimenter avec les 2 raffinats introduits séparément comme décrit dans l'étape B selon l'invention.

## DEFINITIONS & ABREVIATIONS

Dans l'ensemble de la description les termes ou abréviations ci-après ont le sens suivant.

Il est précisé que, dans toute cette description, l'expression « compris(e) entre ... et ...» doit s'entendre comme incluant les bornes citées.

On désigne par l'abréviation EB, l'éthylbenzène.

On désigne par l'abréviation PX, le paraxylène

On désigne par l'abréviation OX, l'orthoxylène.

On désigne par l'abréviation MX, le métaxylène.

On entend par xylènes (XYL), un mélange d'au moins deux isomères choisi parmi l'orthoxylène, le métaxylène, et le paraxylène.

On désigne par l'abréviation anglaise SMB, un lit mobile simulé.

On entend par hydrocarbures en C9+, des hydrocarbures contenant au moins 9 atomes de carbone.

On entend par hydrocarbures en C8+, des hydrocarbures contenant au moins 8 atomes de carbone.

On désigne par C8Aromatiques, encore noté C8A, les hydrocarbures aromatiques contenant 8 atomes de carbone choisi parmi le EB, le PX, le OX, le MX.

On entend par raffinat un mélange de C8A appauvri en PX et pouvant contenir du désorbant, c'est-à-dire présentant une teneur massique en PX inférieur à 2,0%, de préférence inférieur à 1,5% et de manière préférée à 1,0%..

On entend par exempt au sens de la présente invention, une teneur massique en un composé donné par rapport à la masse total de la fraction considérée, par exemple en EB, inférieure à 0,5% poids, de préférence inférieur à 0,1% et de manière préférée inférieure à 0,01%.

On entend par quantité résiduelle d'un composé donné, une quantité dont la teneur massique par rapport à la masse total de la fraction considérée est inférieure à 5,0% poids, de préférence comprise entre 5,0 et 1,0, de préférence comprise entre 4,0 et 1,0, et de manière préférée entre 3,0 et 1,0% poids.

Dans la présente invention, les termes raffinats, effluents, flux et fractions sont employés de façon équivalente.

On entend par colonne de distillation 2 coupes ou 3 coupes, une colonne de distillation permettant l'obtention de 2 ou 3 fractions respectivement.

## PRESENTATION SUCCINCTE DES FIGURES

La figure la représente le schéma général d'une boucle Xylènes mettant en œuvre une étape de séparation par adsorption, une étape de fractionnement, une étape C d'isomérisation en phase vapeur.

La figure 1b représente l'étape B de distillation du raffinat issu de l'étape A selon l'art antérieur.

La figure 2 représente une mise en œuvre du procédé selon l'invention .

## DESCRIPTION DETAILLEE DE L'INVENTION

Les caractéristiques et avantages du procédé selon l'invention, apparaîtront à la lecture de la description ci-après d'exemples non limitatifs de réalisations, en se référant aux figures annexées et décrites ci-après.

Dans le sens de la présente invention, les différents modes de réalisation présentés peuvent être utilisés seuls ou en combinaison les uns avec les autres, sans limitation de combinaison.

La présente invention concerne un procédé d'obtention de paraxylène à partir d'une charge contenant des xylènes, de l'éthylbenzène et des hydrocarbures en C9+, ledit procédé comprenant

- une unique étape A de séparation en lit mobile simulé de ladite charge, ladite étape étant mise en œuvre avec une zéolithe comme adsorbant et un désorbant, à une température comprise entre 20 et 250°C, sous une pression comprise entre la pression de bulle des xylènes à la température opératoire et 2,0 MPa, et avec un rapport volumique du désorbant sur la charge dans l'unité de séparation en lit mobile simulé est compris entre 0,4 et 2,5, et permettant l'obtention d'au moins trois fractions,

   ▪ une fraction A1 comprenant un mélange de paraxylène et de désorbant, et

   ▪ deux fractions A21, A22 comprenant de l'éthylbenzène (EB), de l'orthoxylène (OX) et du métaxylène (MX) et du désorbant,

- une étape B de fractionnement par distillation dans une colonne de distillation des fractions A21 et A22 issue de l'étape A, dans laquelle lesdites fractions sont introduites séparément à des points d'injections distincts et permet l'obtention d'une fraction B2 contenant de l'éthylbenzène, de l'orthoxylène et du métaxylène, et une fraction B42 exempte de composés aromatique contenant 8 atomes de carbones et contenant du désorbant.

Un avantage du procédé selon l'invention est de réaliser un préfractionnement lors de l'étape de séparation en SMB ce qui permet avantageusement de faciliter l'étape de fractionnement du raffinat sans augmenter la complexité du procédé et sans modifier le rendement en Paraxylène.

Un autre avantage du procédé selon l'invention est de pouvoir être mise à profit dans des configurations de dégoulottage (« revamping ») d'une boucle xylènes. En effet, dans ce cas la même colonne de raffinat peut être réutiliser à condition de l'alimenter avec les 2 raffinats introduits séparément comme décrit dans l'étape B selon l'invention.

**Etape A de séparation en lit mobile simulé**

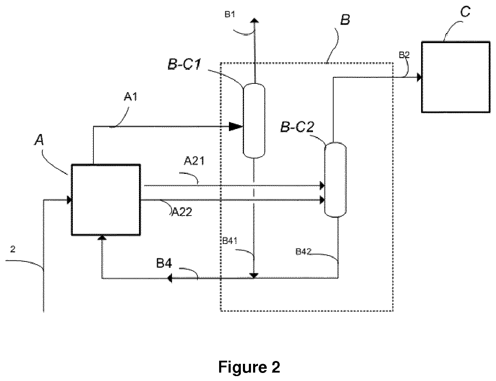

Selon l'invention, le procédé (figure 2) comprend une unique étape A de séparation en lit mobile simulé mise en œuvre avec une zéolithe comme adsorbant et un désorbant et permettant l'obtention au moins trois fractions, une fraction A1 contenant du désorbant et du paraxylène deux fractions A21 et A22, encore appelé « raffinat », appauvri en paraxylène comprenant de préférence constituées, dans des proportions variables d'un mélange de EB, MX, OX, et du désorbant.

Avantageusement, les proportions en EB, MX, OX et désorbant dans les fractions A21 etA22 sont différentes. De préférence, la fraction A21 présente une teneur massique en désorbant inférieure à celle de la fraction A22.

L'étape de séparation de ladite charge est mise en œuvre dans une unité opérant en lit mobile simulé dans au moins une colonne de séparation contenant une pluralité de lits interconnectés et faisant circuler du désorbant en boucle fermée dont sont issus les trois fractions :

- la première est un extrait A1 comprenant du paraxylène et du désorbant, de telles sorte qu'après fractionnement pour éliminer le désorbant le PX atteigne une pureté commerciale de minimum 99,0 % et préférentiellement 99,9 % poids. Avantageusement, l'extrait A1 présente au moins 30% poids de la masse total de l'extrait.

- la fraction A21 appauvrie PX comprend, de préférence est constituée, d'un mélange de EB, de MX, OX, et de désorbant.

- la fraction A22 appauvrie en PX et une quantité résiduelle d'EB et contient un mélange MX, OX, et du désorbant.

De préférence, l'adsorbant utilisé dans l'unité de séparation en lit mobile simulé est une zéolithe X échangée au baryum ou une zéolithe Y échangée au potassium ou une zéolithe Y échangée au baryum et au potassium.

De préférence, le désorbant utilisé dans l'unité de séparation en lit mobile simulé est choisi parmi le paradiéthylbenzène, le toluène, le paradifluorobenzène ou des diéthylbenzènes seul ou en mélange.

De préférence, le rapport volumique du désorbant sur la charge dans l'unité de séparation en lit mobile simulé est compris entre 0,4 et 2,5, de préférence compris entre 0,5 et 2,0 et de manière préférée entre 0,5 et 1,5.

De préférence, l'étape A de séparation en lit mobile simulé est mise en œuvre à une température comprise entre 90 et 210°C, et de manière encore préférée entre 160 et 200°C, et sous une pression comprise entre 1,0 et 2,2 MPa et de préférence comprise entre 1,2 et 2,0 MPa.

De préférence, l'adsorbeur contient une pluralité de lits, interconnectés et répartis sur plusieurs zones délimitées par les injections de la charge et du désorbant , ainsi que des soutirages de l'extrait, et des raffinats.

Selon un mode particulier de réalisation, le nombre total de lits de l'unité de séparation (SMB) est compris entre 10 et 30 lits, et de manière préférée, entre 15 et 18 lits répartis sur un ou plusieurs adsorbeurs, le nombre de lits étant ajusté de manière à ce que chaque lit ait de une hauteur comprise entre 0,70 et 1,40 m.

Selon un mode particulier de réalisation, la répartition de la quantité de solide adsorbant dans chaque zone de l'unité de séparation (SMB) est la suivante :

- la quantité de solide adsorbant en zone 1 est de 18%±8%,

- la quantité de solide adsorbant en zone 2 est de 41%±8%,

- la quantité de solide adsorbant en zone 3A est de 18%±8%,

- la quantité de solide adsorbant en zone 3B est de 14%±8%.

- la quantité de solide adsorbant en zone 4 est de 9%±8%.

Chaque zone délimite les points d'injection et de soutirage, des charge, désorbant, extrait et raffinats comme défini ci-dessous :

o La Zone 1 est comprise entre l'injection du désorbant B4 et le soutirage de l'extrait A1.

o La zone 2 est comprise entre le soutirage de l'extrait A1 et l'injection de la charge

o La zone 3A est comprise entre l'injection de la charge et le soutirage du raffinat A21

o La zone 3B, comprise entre le soutirage du raffinat A21 et le soutirage du raffinat A22

o La zone 4 est comprise entre le soutirage du raffinat A22 et l'injection du désorbant B4

Avantageusement, les performances de l'étape A d'adsorption de la coupe C8A exempte de C9+ sont caractérisées par :

- le taux de récupération du PX dans l'extrait A1 définit par le ratio de PX dans l'extrait A1 / PX dans la charge soit au moins égal à 97,0% et que après récupération du désorbant, dans la colonne d'extrait BC-1 la pureté minimum du PX soit de 99,0% et préférentiellement supérieure à 99,9 %.

La répartition des C8A entre les deux raffinats A21, A22 définie par :

$$R(C8A) = \text{les quantités de C8A dans le raffinat A21 / la quantité de C8A dans le raffinat total A2}$$

soit au minimum de 60% et préférentiellement supérieure à 75%

La répartition du désorbant entre les deux raffinats A21, A22 définie par :

$$R(\text{Désorbant}) = \text{la quantité de désorbant dans le raffinat A21/ la quantité de désorbant dans le raffinat total A2}$$

le paramètre de séparation « delta R » définit par l'écart de répartition R(C8A)- R(Désorbant) .

De préférence, le paramètre de séparation minimum est de 10%, préférentiellement de 20% et plus préférentiellement de 30%.

Ainsi l'étape d'adsorption A de la charge permet de séparer le PX, et également de préfractionner le raffinat A2 en deux flux l'un A21, enrichi en C8A, et l'autre A22 enrichi en désorbant.

**Etape B de fractionnement**

Le procédé selon l'invention comprend une étape B de fractionnement par distillation dans une colonne des fractions A21 et A22 issues de l'étape A de séparation.

Selon l'invention, lesdites fractions A21 et A22 sont introduites séparément dans la colonne de raffinat B-C2 à des points d'injections distincts.

Dans un mode particulier de l'invention où le désorbant utilisé dans l'étape A est lourd, c'est-à-dire avec un point d'ébullition supérieur à celui des C8A, la fraction dont la teneur en désorbant lourd est la plus élevée est introduite quelques plateaux en dessous de la position de l'alimentation de la fraction dont la teneur en désorbant lourd est la plus faible.

Avantageusement, l'étape B permet de produire en tête de colonne une fraction B2 exempte de désorbant et appauvrie de PX et contenant le MX, OX et l'éthylbenzène et en fond une fraction B42 exempte de C8A et constitué de désorbant.

Un avantage de cette double alimentation est de permettre de réduire la charge thermique de la colonne de raffinat d'au minimum de 2,0 à 15,0 %, selon la qualité du préfractionnement des raffinats obtenue à l'étape A.

Cette avantage peut être obtenu pour tout type de désorbant dans la mesure où ladite étape A est mise en œuvre de telle sorte à effectuer un préfractionnement en deux raffinats A21 et A22 l'un enrichi en désorbant, l'autre enrichi en C8A (OX, MX, EB).

Dans un mode particulier de l'invention où le désorbant utilisé dans l'étape A est léger, c'est-à-dire avec un point d'ébullition inférieur à celui des C8A, la fraction du raffinat dont la teneur en désorbant léger est la plus élevée est introduite quelques plateaux au-dessus de la position de l'alimentation du raffinat dont la teneur en désorbant léger est la plus faible.

Avantageusement, l'étape B permet de produire en fond de colonne une fraction B2 exempte de désorbant et appauvrie en PX et contenant le MX,OX et l'éthylbenzène et en tête une fractionB42 exempte de C8A et constitué de désorbant.

De préférence, la colonne de distillation mise en œuvre est choisi parmi une colonne 2 coupes et une colonne 3 coupes.

Avantageusement, ladite colonne présente un nombre de plateaux théoriques compris entre 30 et 80, de préférence entre 35 et 75, de manière préférée 40 et 70, de manière très préférée entre 45 et 65.

De préférence, les deux points d'injections des fractions A21 et A22 dans la colonne de distillation présente un écartement compris entre 2 et 15 plateaux théoriques, de préférence entre 3 et 12 plateaux théoriques et plus préférentiellement entre 4 et 9.

Dans un mode de réalisation préféré, la colonne de distillation mise en œuvre est une colonne 2 coupes.

Avantageusement, ladite colonne 2 coupes présente un nombre de plateaux théoriques compris entre 30 et 70, de préférence entre 35 et 65, de manière préférée 40 et 60, de manière très préférée entre 45 et 55.

Sans être lié à aucune théorie, il a été découvert que, pour minimiser la charge thermique de la colonne de raffinat cet écartement est corrélé à qualité du préfractionnement des raffinats opéré dans l'étape A. De préférence afin de minimiser la charge thermique de la colonne B-C2 lorsque la qualité du préfractionnement varie, l'injection de la fraction A22 se fera par un système de distribution sur différents plateaux de telle sorte que l'écartement entre les points d'injection des charges A21 et A22 augmente lorsque le paramètre de séparation R augmente. Ladite colonne 2 coupes comprend en outre un condenseur et un rebouilleur, opérée à 0,2 MPa avec un taux de reflux de 1,2.

Dans un mode de réalisation particulier, lorsque le désorbant mis en œuvre à l'étape A est un composé plus lourd que les xylènes, c'est-à-dire présentant un point d'ébullition plus élevé que celui des xylènes, le raffinat enrichi en désorbant, c'est à dire la fraction A22, est introduit en dessous du raffinat appauvri en désorbant, c'est à dire la fraction A21.

Dans un autre mode de réalisation, lorsque le désorbant de ladite étape de séparation est un composé plus léger que les xylènes, c'est-à-dire présentant un point d'ébullition plus faible que celui des xylènes, le raffinat enrichi en désorbant, c'est à dire la fraction A21, est introduit au-dessus du raffinat appauvri en désorbant, c'est à dire la fraction A22.

Dans un autre mode de réalisation, la colonne de distillation mise en œuvre est une colonne 3 coupes. De préférence, ladite colonne comprend une paroi interne placée, de préférence, dans la zone de rectification et permet de recueillir deux fractions B2 et B3 exemptes de désorbant et une fraction B42 exempte de C8A et comprenant, de préférence constitué, de désorbant.

De préférence, l'injection des fractions A21 et A22 est réalisée de part et d'autre de la paroi interne. En d'autres termes, avantageusement, les positions des deux alimentations des fractions A21 et A22 se situent de part et d'autre de la paroi interne.

De préférence, la fraction A1 issue de l'étape A contenant, et de préférence constituée, d'un mélange de PX et de désorbant est engagée dans une étape de fractionnement par distillation dans une colonne de distillation (B-C1) permettant l'obtention d'une fraction B1 exempte de désorbant constituée de PX et une fraction B41 constituée de désorbant. Ladite distillation est mise en œuvre selon les connaissances de l'Homme du métier.

Avantageusement, les fractions désorbant B41 et B42 exemptes de C8A sont récupérées en fond de chaque colonne de distillation, lorsque le désorbant est lourd et en tête lorsque le désorbant est léger. Lesdites fractions sont ensuite mélangées et renvoyées vers l'étape A d'adsorption en lit mobile simulé par l'intermédiaire du flux B4.

**Etape** C d'isomérisation en phase vapeur

Le procédé comprend en outre une étape C d'isomérisation en phase vapeur de la fraction B2 comprenant de l'éthylbenzène, de l'orthoxylène et du métaxylène issu de l'étape B de fractionnement.

Avantageusement l'étape C d'isomérisation en phase vapeur permet l'isomérisation des xylènes ainsi que de l'EB, dans une unité opérant en phase vapeur, à haute température et convertissant l'éthylbenzène en xylènes, pour traiter le raffinat B2 riche en EB issu de l'étape B.

L'étape d'isomérisation en phase vapeur permet de convertir l'EB en xylènes avec un taux de conversion par passe de l'éthylbenzène généralement comprise entre 10 et 50%, de préférence comprise entre 20 et 40%, avec une perte en C8 Aromatiques (C8A) inférieure à 5,0 %poids, de préférence inférieure à 3,0 % poids et préférentiellement inférieure à 1,8 % poids.

Avantageusement ladite étape C permet également d'isomériser les xylènes, de telle sorte que le paraxylène (PX) ait une concentration à l'équilibre thermodynamique; définie par

$$(C_{eff}-C_{in}) \times 100 / (C_{eq} - C_{in})$$

dans lesquels

Ceff et Cin sont les concentrations en PX respectivement dans la coupe C8A de l'effluent et de la charge du réacteur d'isomérisation,

Ceq est la concentration à l'équilibre thermodynamique du PX dans la coupe C8A à la température réactionnelle ; supérieure ou égale à 90%.

L'étape d'isomérisation en phase vapeur est mise en œuvre à une température supérieure à 300°C, de préférence comprise entre 350 et 480°C, une pression inférieure à 4,0 MPa, de préférence comprise entre 0,5 et 2,0 MPa, une vitesse spatiale inférieure à 10,0 h$^{-1}$, de préférence comprise entre 0,5 et 6,0 h$^{-1}$, rapport molaire hydrogène sur hydrocarbure inférieur à 10,0, de préférence compris entre 3,0 et 6,0, et en présence d'un catalyseur comportant au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids.

Tous les catalyseurs susceptibles d'isomériser les hydrocarbures à 8 atomes de carbone, zéolitiques ou non, conviennent pour l'unité d'isomérisation en phase vapeur. De préférence, on utilise un catalyseur contenant une zéolithe acide, par exemple de type structural MFI, MOR, MAZ, FAU et/ou EUO. De manière encore plus préférée, on utilise un catalyseur contenant une zéolithe de type structural EUO et au moins un métal du groupe VIII de la classification périodique des éléments.

Selon une variante préférée du procédé, le catalyseur utilisé à l'étape C comprend de 1 à 70% poids d'une zéolithe de type structural EUO, de préférence EU-1, comprenant du silicium et au moins un élément T choisi de préférence parmi l'aluminium et le bore dont le rapport Si/T est compris entre 5 et 100.

De préférence, la zéolithe est sous forme hydrogène au moins en partie, et la teneur en sodium est telle que le ratio atomique Na/T est inférieur à 0,1.

De préférence, le catalyseur comprend entre 0,01 et 2% poids d'étain ou d'indium, et du soufre à raison de 0,5 à 2 atomes par atome de métal du groupe VIII.

L'effluent C1 obtenu à l'étape C, présentant des concentrations en isomère PX, OX et MX proches des concentrations de l'équilibre thermodynamique sont recyclés vers l'étape A d'adsorption en lit mobile simulé.

Dans un mode de réalisation particulier, lorsque l'effluent C1 contient des composés lourds et légers formés par les réactions indésirables ledit effluent est alors engagé dans une étape optionnelle de fractionnement pour éliminer lesdits composés.

## EXEMPLES

Les exemples ci-dessous illustrent l'invention sans en limiter la portée.

**Exemple 1 :**

Cet exemple montre l'intérêt de l'invention en comparant les performances de l'étape de distillation B placée entre une étape d'adsorption A et une étape d'isomérisation C, lesdites étapes faisant partie d'un complexe aromatique produisant du paraxylène à partir d'un reformat.

Dans cet exemple on considère 543 t/h d'une coupe C8 issue d'une colonne de xylène et comprenant des C8Aromatiques (C8A) provenant d'un réformat, d'une unité de transalkylation et d'une ou plusieurs unités d'isomérisation, sa composition est en % poids :

| EB | 4,1% |
|------|-------|
| PX | 23,1% |
| MX | 50,3% |
| OX | 22,4% |
| C9+ | 0,1% |

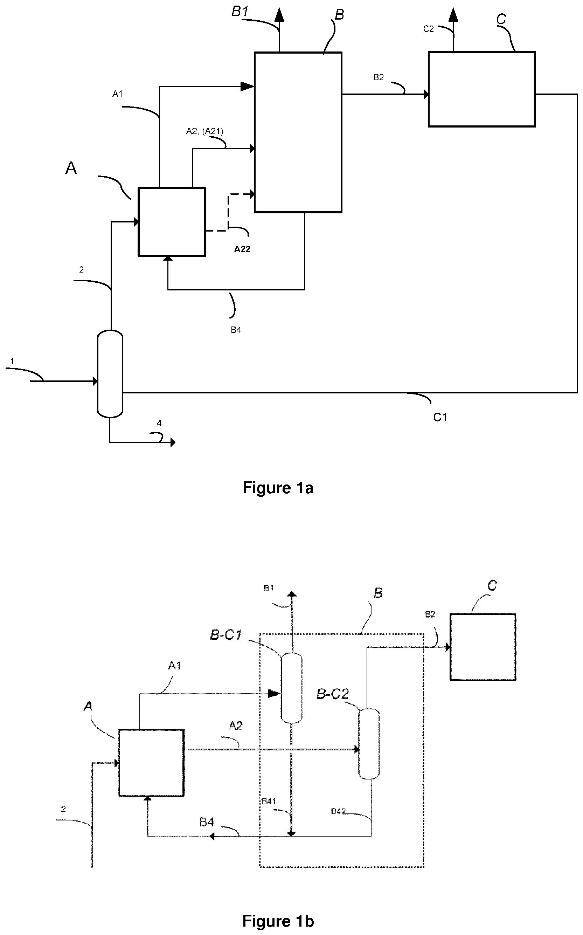

Selon l'état de l'art représenté sur la figure 1b), cette coupe C8A est envoyée dans une étape A d'adsorption en lit mobile simulé comprenant un adsorbeur avec 4 zones délimitées par les injections de charges et de désorbant B4 et les soutirages de raffinat A2 et d'extrait A1. Cet adsorbeur est composé de 15 lits contenant de la zéolithe X échangée au baryum répartis comme suit :

◦ 3 lit en Zone 1, comprise entre l'injection du désorbant B4 et le soutirage de l'extrait A1.

◦ 6 lits en zone 2 , comprise entre le soutirage de l'extrait A1 et l'injection de la charge

◦ 4 lits en zone 3, comprise entre l'injection de la charge et le soutirage du raffinat A2

◦ 2 lits en zone 4, comprise entre le soutirage du raffinat A2 et l'injection du désorbant B4

La température est de 175 °C. Le désorbant utilisé est le Paradiéthylbenzène, le taux de solvant par rapport à la charge est de 1,2 (vol/vol).

Ainsi mise en œuvre l'unité de séparation par adsorption A permet de produire 2 flux A1 et A2 alimentant l'étape de distillation B :

*Un extrait A1 contenant au moins 97 % du PX de la charge et une partie du désorbant, lequel est envoyé dans une colonne d'extrait B-C1 afin de récupérer le PX pur en tête (flux B1) et l'adsorbant en fond (flux B41).

*827,9 t/h d'un raffinat A2 substantiellement exempt de PX, contenant 407,1 t/h de désorbant

le raffinat A2 est alimenté au plateau théorique 25 dans la colonne de distillation B-C2 contenant 47 plateaux théoriques, un condenseur et un rebouilleur, opérée à 0,2 MPa avec un taux de reflux de 1,4-. Cette colonne permet de produire 2 flux : 420 t/h d'un raffinat en tête B2 contenant 25 ppm de désorbant et de 407 t/h de désorbant B42 en fond contenant 50ppm de xylènes et renvoyé au lit mobile simulé, après mélange avec le flux B41, et échange thermique à la température requise pour l'adsorption. Le fractionnement du raffinat A2 par la colonne de raffinat décrit ainsi nécessite 80 Gcal/h d'énergie de rebouillage. Le raffinat B2 est envoyé vers la première étape d'isomérisation (C).

Dans une mise en œuvre du procédé selon l'invention représenté sur la figure 2), ladite coupe C8A est envoyée dans une étape A d'adsorption en lit mobile simulé comprenant un adsorbeur avec 5 zones délimitées par les injections de charges et de désorbant (B4) et les soutirages des raffinats A21, A22 et d'extrait A1. Ledit adsorbeur est composé de 18 lits contenant de la zéolithe X échangée au baryum répartis comme suit :

o 3 lit en Zone 1, comprise entre l'injection du désorbant B4 et le soutirage de l'extrait A1,

◦ 6 lits en zone 2 , comprise entre le soutirage de l'extrait A1 et l'injection de la charge,

◦ 4 lits en zone 3A, comprise entre l'injection de la charge et le soutirage du raffinat A21,

◦ 3 lits en zone 3B, comprise entre le soutirage du raffinat A21 et le soutirage du raffinat A22,

◦ 2 lits en zone 4, comprise entre le soutirage du raffinat A22 et l'injection du désorbant B4.

La température est de 175 °C. Le désorbant utilisé est le Paradiéthylbenzène, le taux de solvant par rapport à la charge est de 1,2 (vol/vol).

Ainsi la mise en œuvre à l'étape A de l'unité de séparation par adsorption selon l'invention permet d'obtenir trois fractions A1, A21, et A22, selon la répartition suivante.

- Un extrait A1 contenant au moins 97 % du paraxylène PX de la charge et une partie du désorbant, lequel est envoyé dans une colonne d'extrait afin de récupérer le PX pur en tête et le désorbant en fond.

- 507,5t/h de raffinat léger A21 et

- 320,4 t/h de raffinat lourd A22.

Les raffinats A21 et A22 sont soutirés de part et d'autre de la zone 3B de l'unité A d'adsorption en lit mobile simulé, et présentent les compositions suivantes :

- • R(C8A), la quantités de C8A dans le raffinat A21 / la quantité de C8A dans le raffinat total = 79%

- • R(Désorbant), la quantité de désorbant dans le raffinat A21/ la quantité de désorbant dans le raffinat total = 43%

- • Le paramètre de séparation Delta R = 36%

Lors de l'étape B de fractionnement par la colonne de distillation B-C2 contenant 47 plateaux théoriques, le raffinat A21 est alimenté au plateau théorique 24, et le raffinat A22 est alimenté au plateau théorique 30. Ladite colonne comprend en outre un condenseur et un rebouilleur, opérée à 0,2 MPa avec un taux de reflux de 1,2. Ladite colonne permet de produire les 2 fractions suivantes :

- - 420 t/h d'un raffinat en tête B2 contenant du MX, OX, EB et 25 ppm de désorbant et

- - de 407 t/h de désorbant B42 en fond contenant 50ppm de xylènes et renvoyé au lit mobile simulé, après mélange avec le flux B41, et échange thermique à la température requise pour l'adsorption. Le fractionnement des raffinats A21 et A22 par la colonne de raffinat décrit ainsi nécessite 74,4 Gcal/h d'énergie de rebouillage. Le raffinat B2 est envoyé vers l'étape C d'isomérisation.

Cet exemple illustre clairement que l'obtention d'un raffinat enrichi en désorbant et d'un raffinat enrichi en MX, OX, EB par la mise en œuvre dans une étape A de séparation d'une unité d'adsorption en lit mobile simulé, associée à leur introduction séparée dans une colonne de distillation permet de faciliter leur fractionnement et ainsi d'alléger sa charge thermique.
Contrairement au procédé décrit dans l'état de l'art où la colonne de raffinat n'est alimentée que par une charge, il est, conformément au procédé selon l'invention, possible de réduire la charge thermique du rebouilleur de 7%, sans ajouter d'autre étapes ou autre équipement au complexe aromatique connu dans l'état de l'art.

**Exemple 2 :**

Cet exemple illustre l'intérêt de l'invention, lorsque les performances du préfractionnement du raffinat lors de l'étape A d'adsorption varient, comme cela peut arriver par exemple au cours d'un changement de réglage des paramètres opératoires de l'adsorbeur ou d'un changement de tamis moléculaire. Les conditions de cet exemple sont identiques à celles de l'exemple 1. on simule une variation du paramètre de séparation obtenu via un changement de tamis. La position de l'alimentation du raffinat léger A21 dans la colonne de raffinat n'est pas impactée, celle du raffinat lourd est très légèrement modifiée, le gain de charge thermique du rebouilleur augmente significativement avec la qualité du préfractionnement du raffinat.
L'amélioration des capacités et des sélectivités d'adsorption du tamis moléculaire peuvent être mises à profit pour réduire la consommation énergétique du complexe aromatique sans modification de la configuration du complexe aromatique ni de ses performances en terme de productivité.

|  | état de l'art | cas 1 | cas 2 | cas 3 |
|---|---|---|---|---|
| R(C8A) | 1 | 0,78 | 0,78 | 0,88 |
| R(Désorbant), | 1 | 0,43 | 0,33 | 0,33 |
| Paramètre de séparation Delta R | 0 | 0,36 | 0,45 | 0,55 |
| Position de l'alimentation A2 | 25 | NA | NA | NA |
| Position de l'alimentation A22 | NA | 29 | 30 | 31 |
| Position de l'alimentation A21 | NA | 24 | 24 | 24 |
| Charge thermique du rebouilleur (Gcal/h) | 80,0 | 74,4 | 72,6 | 71,0 |
| Gain énergétique |  | 7% | 9% | 11% |

**Revendications**

1. Un procédé d'obtention de paraxylène à partir d'une charge contenant des xylènes, de l'éthylbenzène et des hydrocarbures en C9+, ledit procédé comprenant

    - une unique étape A de séparation en lit mobile simulé de ladite charge, ladite étape étant mise en œuvre avec une zéolithe comme adsorbant et un désorbant, à une température comprise entre 20 et 250°C, sous une pression comprise entre la pression de bulle des xylènes à la température opératoire et 2,0 MPa, et avec un rapport volumique du désorbant sur la charge dans l'unité de séparation en lit mobile simulé est compris entre 0,4 et 2,5, et permettant l'obtention d'au moins trois fractions,

- une fraction A1 comprenant un mélange de paraxylène et de désorbant, et
- deux fractions A21, A22 comprenant de l'éthylbenzène (EB), de l'orthoxylène (OX) et du métaxylène (MX) et du désorbant,

- une étape B de fractionnement par distillation dans une colonne de distillation des fractions A21 et A22 issue de l'étape A, dans laquelle lesdites fractions sont introduites séparément à des points d'injections distincts et permet l'obtention d'une fraction B2 contenant de l'éthylbenzène, de l'orthoxylène et du métaxylène, et une fraction B42 exempte de composés aromatique contenant 8 atomes de carbones et contenant du désorbant.

2. Procédé selon la revendication 1 dans lequel la colonne de distillation mise en œuvre à l'étape B présente un nombre de plateaux théoriques compris entre 30 et 80, de préférence entre 35 et 75, de manière préférée 40 et 70, de manière très préférée entre 45 et 65.

3. Procédé selon l'une quelconque des revendications précédentes dans lequel les points injections des fractions A21 et A22 dans la colonne de distillation mise en œuvre à l'étape B présente un écartement compris entre 2 et 15 plateaux théoriques, de préférence entre 3 et 12.

4. Procédé selon l'une quelconque des revendications 1 à 3 dans lequel la colonne de distillation mise en œuvre à l'étape B est choisie parmi une colonne 2 coupes et une colonne 3 coupes.

5. Procédé selon l'une quelconque des revendications précédentes dans lequel la fraction A21 présente une teneur massique en désorbant inférieure à celle de la fraction A22.

6. Procédé selon l'une quelconque des revendications précédentes dans lequel l'étape A de séparation en lit mobile simulé est mise en œuvre à une température comprise 90 et 210°C, et sous une pression comprise entre 1,0 et 2,2 MPa.

7. Procédé selon l'une quelconque des revendications précédentes dans lequel le nombre total de lits de l'unité de séparation (SMB) mis en œuvre à l'étape A est compris entre 10 et 30 lits, et de manière préférée, entre 15 et 18 lits.

8. Procédé selon l'une quelconque des revendications 5 à 7 dans lequel le désorbant mis en œuvre à l'étape A est un composé présentant un point d'ébullition plus élevé que celui des xylènes, et la fraction A22 est introduite en dessous de la fraction A21.

9. Procédé selon l'une quelconque des revendications 5 à 7 dans lequel le désorbant mis en œuvre à l'étape A est un composé présentant un point d'ébullition plus faible que celles des xylènes, et la fraction A22 est introduite au-dessus de la A21.

10. Procédé selon l'une quelconque des revendications précédentes comprenant une étape C d'isomérisation en phase vapeur de la fraction B2 comprenant de l'éthylbenzène, de l'orthoxylène et du métaxylène issu de l'étape B de fractionnement.

11. Procédé selon la revendication 10 dans lequel l'étape d'isomérisation est mise en œuvre à une température supérieure à 300°C, de préférence comprise entre 350 et 480°C, une pression inférieure à 4,0 MPa, de préférence comprise entre 0,5 et 2,0 MPa, une vitesse spatiale inférieure à 10,0 h-1, de préférence comprise entre 0,5 et 6,0 $h^{-1}$, rapport molaire hydrogène sur hydrocarbure inférieur à 10,0, de préférence compris entre 3,0 et 6,0, et en présence d'un catalyseur comportant au moins une zéolithe présentant des canaux dont l'ouverture est définie par un anneau à 10 ou 12 atomes d'oxygène (10 MR ou 12 MR), et au moins un métal du groupe VIII de teneur comprise entre 0,1 et 0,3% poids.

**Patentansprüche**

1.  Verfahren zum Erhalten von para-Xylol ausgehend von einer Charge, die Xylole, Ethylbenzol und Kohlenwasserstoffe des Typs C9+ enthält, wobei das Verfahren Folgendes umfasst,

    - einen einzigen Schritt A, in welchem die Charge in einem simulierten Fließbett aufgetrennt wird, wobei der Schritt mit einem Zeolith als Adsorptionsmittel und einem Desorptionsmittel, bei einer Temperatur im Bereich von 20 bis 250 °C, bei einem Druck im Bereich vom Blasenbildungsdruck der Xylole bei der Betriebstemperatur bis zu 2,0 MPa, und mit einem Volumenverhältnis des Desorptionsmittels zur Charge in der Einheit zum Auftrennen in einem simulierten Fließbett im Bereich von 0,4 bis 2,5 durchgeführt wird, und wobei er es ermöglicht, mindestens drei Fraktionen zu erhalten,

    • eine Fraktion A1, die eine Mischung aus para-Xylol und Desorptionsmittel umfasst, und
    • zwei Fraktionen A21, A22, die Ethylbenzol (EB), ortho-Xylol (OX) und meta-Xylol (MX) sowie Desorptionsmittel umfassen,

    - einen Schritt B der Fraktionierung, mittels Destillation in einer Destillationskolonne, der Fraktionen A21 und A22, welche aus dem Schritt A stammen, wobei die Fraktionen getrennt voneinander an gesonderten Einleitungsstellen eingeleitet werden, wobei auf diese Weise eine Fraktion B2, welche Ethylbenzol, ortho-Xylol und meta-Xylol enthält, und eine Fraktion B42, welche frei von aromatischen Verbindungen ist, die 8 Kohlenstoffatome enthalten, und welche Desorptionsmittel enthält, erhalten werden können.

2.  Verfahren nach Anspruch 1, wobei die Destillationskolonne, welche im Schritt B eingesetzt wird, eine Anzahl an theoretischen Böden aufweist, die im Bereich von 30 bis 80, vorzugsweise von 35 bis 75, auf bevorzugte Weise 40 bis 70, mit besonderem Vorzug von 45 bis 65 liegt.

3.  Verfahren nach einem beliebigen der vorhergehenden Ansprüche, wobei die Einleitungsstellen der Fraktionen A21 und A22 in die Destillationskolonne, welche im Schritt B eingesetzt wird, eine Beabstandung im Bereich von 2 bis 15, vorzugsweise von 3 bis 12, theoretischen Böden aufweisen.

4.  Verfahren nach einem beliebigen der Ansprüche 1 bis 3, wobei die Destillationskolonne, welche im Schritt B eingesetzt wird, aus einer 2-Fraktionen-Kolonnen und einer 3-Fraktionen-Kolonne ausgewählt ist.

5.  Verfahren nach einem beliebigen der vorhergehenden Ansprüche, wobei die Fraktion A21 einen massenbezogenen Gehalt an Desorptionsmittel aufweist, welcher geringer als derjenige der Fraktion A22 ist.

6.  Verfahren nach einem beliebigen der vorhergehenden Ansprüche, wobei der Schritt A des Auftrennens im simulierten Fließbett bei einer Temperatur im Bereich von 90 bis 210 °C und bei einem Druck im Bereich von 1,0 bis 2,2 MPa durchgeführt wird.

7.  Verfahren nach einem beliebigen der vorhergehenden Ansprüche, wobei die Gesamtzahl an Betten der Auftrennungseinheit (SMB), die im Schritt A eingesetzt werden, im Bereich von 10 bis 30 Betten, vorzugsweise von 15 bis 18 Betten liegt.

8.  Verfahren nach einem beliebigen der Ansprüche 5 bis 7, wobei es sich bei dem Desorptionsmittel, welches im Schritt A eingesetzt wird, um eine Verbindung handelt, die einen Siedepunkt aufweist, welcher höher als derjenige der Xylole ist, und die Fraktion A22 unterhalb der Fraktion A21 eingeleitet wird.

9.  Verfahren nach einem beliebigen der Ansprüche 5 bis 7, wobei es sich bei dem Desorptionsmittel, welches im Schritt A eingesetzt wird, um eine Verbindung handelt, die einen Siedepunkt aufweist, welcher niedriger als derjenige der Xylole ist, und die Fraktion A22 oberhalb der A21 eingeleitet wird.

10. Verfahren nach einem beliebigen der vorhergehenden Ansprüche, wobei es einen Schritt C umfasst, in welchem die Fraktion B2, welche Ethylbenzol, ortho-Xylol umfasst, sowie meta-Xylol, welches aus dem Fraktionierungsschritt B stammt, in der Gasphase isomerisiert werden.

11. Verfahren nach Anspruch 10, wobei der Isomerisierungsschritt bei einer Temperatur, die mehr als 300 °C beträgt, wobei sie vorzugsweise im Bereich von 350 bis 480 °C liegt, einem Druck von weniger als 4,0 MPa, wobei vorzugsweise im Bereich von 0,5 bis 2,0 MPa liegt, einer Raumgeschwindigkeit von weniger als 10,0 h$^{-1}$, wobei sie vorzugsweise im Bereich von 0,5 bis 6,0 h$^{-1}$ liegt, einem Molverhältnis von Wasserstoff zu Kohlenwasserstoffen von weniger als 10,0, wobei es vorzugsweise im Bereich von 3,0 bis 6,0 liegt, sowie in Gegenwart eines Katalysators durchgeführt wird, welcher mindestens einen Zeolith, der mit Kanälen versehen ist, deren Öffnung durch einen Ring mit 10 oder 12 Sauerstoffatomen (10 MR oder 12 MR) begrenzt ist, und mindestens ein Metall der Gruppe VIII in einem Gehalt im Bereich von 0,1 bis 0,3 Gewichts-% aufweist.

**Claims**

1. Process for obtaining para-xylene from a feedstock containing xylenes, ethylbenzene and C9+ hydrocarbons, said process comprising

   - a single stage A of separation in a simulated moving bed of said feedstock, said stage being carried out with a zeolite as adsorbent and a desorbent, at a temperature between 20 and 250°C, under a pressure between the bubble pressure of the xylenes at the operating temperature and 2.0 MPa, and with a ratio by volume of the desorbent to the feedstock in the unit for separation in a simulated moving bed between 0.4 and 2.5, and making it possible to obtain at least three fractions,

      □ a fraction A1 comprising a mixture of para-xylene and of desorbent, and
      □ two fractions A21, A22 comprising ethylbenzene (EB), ortho-xylene (OX) and meta-xylene (MX) and desorbent,

   - a stage B of fractionation by distillation in a distillation column of the fractions A21 and A22 resulting from stage A, in which said fractions are introduced separately at distinct injection points, and makes it possible to obtain a fraction B2 containing ethylbenzene, ortho-xylene and meta-xylene, and a fraction B42 devoid of aromatic compounds containing 8 carbon atoms and containing desorbent.

2. Process according to Claim 1, in which the distillation column employed in stage B exhibits a number of theoretical plates between 30 and 80, preferably between 35 and 75, in a preferred way 40 and 70, very preferably between 45 and 65.

3. Process according to either one of the preceding claims, in which the injection points of the fractions A21 and A22 into the distillation column employed in stage B exhibits a spacing between 2 and 15 theoretical plates, preferably between 3 and 12.

4. Process according to any one of Claims 1 to 3, in which the distillation column employed in stage B is chosen from a 2-cut column and a 3-cut column.

5. Process according to any one of the preceding claims, in which the fraction A21 exhibits a content by weight of desorbent which is lower than that of the fraction A22.

6. Process according to any one of the preceding claims, in which stage A of separation in a simulated moving bed is carried out at a temperature between 90 and 210°C, and under a pressure between 1.0 and 2.2 MPa.

7. Process according to any one of the preceding claims, in which the total number of beds of the separation unit (SMB) employed in stage A is between 10 and 30 beds, and in a preferred way between 15 and 18 beds.

8. Process according to any one of Claims 5 to 7, in which the desorbent employed in stage A is a compound exhibiting a higher boiling point than that of the xylenes, and the fraction A22 is introduced below the fraction A21.

9. Process according to any one of Claims 5 to 7, in which the desorbent employed in stage A is a compound exhibiting a lower boiling point than that of the xylenes, and the fraction A22 is introduced above the A21.

10. Process according to any one of the preceding claims, comprising a stage C of vapor-phase isomerization of the fraction B2 comprising ethylbenzene, ortho-xylene and meta-xylene resulting from the fractionation stage B.

11. Process according to Claim 10, in which the isomerization stage is carried out at a temperature of greater than 300°C, preferably between 350 and 480°C, a pressure of less than 4.0 MPa, preferably between 0.5 and 2.0 MPa, a space velocity of less than 10.0 h$^{-1}$, preferably between 0.5 and 6.0 h$^{-1}$, a hydrogen to hydrocarbon molar ratio of less than 10.0, preferably between 3.0 and 6.0, and in the presence of a catalyst comprising at least one zeolite exhibiting channels, the opening of which is defined by a ring having 10 or 12 oxygen atoms (10 MR or 12 MR), and at least one metal from Group VIII with a content between 0.1% and 0.3% by weight.

Figure 1a

Figure 1b

**Figure 2**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2862638
- FR 2844790
- FR 2922547